# EUROPEAN PATENT APPLICATION

(11) **EP 4 174 623 A1**
(43) Date of publication of application: **03.05.2023**
(21) Application number: 22203706.1
(22) Date of filing: 26.10.2022
(51) Int. Cl.: G06F 3/01, G02B 27/00, H04N 23/60, A61B 34/00

(54) **AUTOMATED USER PREFERENCES**

(30) Priority: 26.10.2021 BE 202105836
(71) Applicant: Rods & Cones Holding BV, 1114 AN Amsterdam-Duivendrecht (NL)
(72) Inventor: QUIRYNEN, Benoit, 1114 AN Amsterdam-Duivendrecht (NL); DHEEDENE, Jan, 1114 AN Amsterdam-Duivendrecht (NL); DHEEDENE, Bruno, 1114 AN Amsterdam-Duivendrecht (NL); VULJANIC, Dario, 1114 AN Amsterdam-Duivendrecht (NL); KOFJAC, Dalibor, 1114 AN Amsterdam-Duivendrecht (NL)
(74) Representative: Brantsandpatents bv

(57) **Abstract**

The present invention relates to a method of applying automated user preferences on head-mounted devices, such as smartglasses, as well as a head-mounted device configured for this purpose, and the use thereof.

## Description

### TECHNICAL FIELD

The invention relates to head-mounted devices with an image sensor or camera, such as smartglasses, headsets with a camera, but also headgear on which a (detachable or not) camera or camera module is provided. Specifically, the invention relates to the storage of user preferences for the image sensor on the head-mounted device, and the automatic recognition of situations with associated preferences and automatic loading of these preferences, such as during use in a surgical operation, but also in other processes where a user should not or cannot use their hands to change these settings.

### PRIOR ART

In the prior art, head-mounted image sensor devices, such as, for example, smartglasses, are increasingly being used to perform sensitive operations, sometimes with external guidance. These systems have one or more image sensors (cameras), which have become more sophisticated over the years and have gained many functionalities. Thus, these image sensors can operate under a very wide range of operational parameters, such as focus distance, shutter speed, resolution (image quality), color balance, lighting support, etc.

In these very diverse applications, it is therefore necessary to have a flexible image sensor, which can easily, quickly, and accurately capture images in different configurations (exposure, focal length, resolution, etc.). Due to the additional difficulty that in some situations it is impossible to use the hands for this, or very disadvantageous (endangering sterility during an operation), other solutions must be sought. For example, it is possible to work via voice control, but this is often subject to other problems (again with the example of a medical procedure, mouth masks are often used, which can distort or weaken the voice and the system may not recognize it). Moreover, in such cases it is also necessary to know all the correct "signals" beforehand, as each system typically has a different trigger to perform certain actions.

An example of a known system in the prior art is described in WO2020/084625. It however makes use of external camera systems, for which presets are defined coupled to smartglasses.

The present invention aims to find a solution for at least some of the above problems.

### SUMMARY OF THE INVENTION

The invention relates to an improved method of using a head-mounted device in an activity, particularly wherein the device is configured to recognize certain routines or postures, and automatically load and set an appropriate set of parameters for the device.

In particular, the invention relates to a method for this purpose according to any of claims 1-13.

In a second aspect, the invention relates to a head-mounted device configured for performing the method according to the first aspect of the invention.

In a second aspect, the invention relates to a use of a head-mounted device according to the second aspect in a medical action with the method of the first aspect, by a medical professional.

Additional possible uses are repair work in hard-to-reach locations (work at height, remote places, etc.), wherein users are not required to fully master all aspects of their task.

In a third aspect, the invention relates to the use of a head-mounted device according to the second aspect of the invention, configured to perform the method according to the first aspect of the invention.

### DESCRIPTION OF THE FIGURES

**Figure 1** shows an embodiment of a head-mounted device according to the invention in the form of smartglasses with a display, with all components provided on the head-mounted device.
**Figure 2** shows an embodiment of a head-mounted device according to the invention in the form of smartglasses with a display, and an external control unit wiredly connected to the smartglasses.
**Figure 3** shows an embodiment of a head-mounted device according to the invention in the form of smartglasses with a display, with all components provided on the head-mounted device, and an external control unit wirelessly connected to the smart glasses.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

When the term "around" or "about" is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term "about" or "around" is used, is itself specifically disclosed.

The terms "comprise", "comprising", "consist of", "consisting of", "provided with", "have", "having", "include", "including", "contain", "containing" are synonyms and are inclusive or open terms that indicate the presence of what follows, and which do not exclude or prevent the presence of other components, characteristics, elements, members, steps, as known from or disclosed in the prior art.

The term "image sensor" refers to any electrical or electronic component capable of capturing information that can be used for imaging, such as CCD or CMOS sensors, and typically refers to a camera (digital or analog).

In a first aspect, the invention relates to a method of using a head-mounted device in an activity, the head-mounted device comprising at least a sensor system for detecting the three-dimensional orientation of the head-mounted device, an image sensor for capturing images, a memory element, and a processor for controlling the image sensor, the sensor system and the memory element,
the method comprising the following steps:
a. initiating a session of the activity;
b. setting and confirming during the session one or more parameters under which the image sensor operates;
c. storing on the memory element a set of parameters under which the image sensor operates, comprising the set and confirmed one or more parameters, wherein the set of parameters stored is associated with the orientation, preferably the three-dimensional orientation, of the head-mounted device when confirming and/or storing the set of parameters;
d. upon recognizing a (preferably three-dimensional) orientation of the head-mounted device as an (preferably three-dimensional) orientation for which an associated set of parameters is stored, automatically loading and setting the parameters associated with the recognized three-dimensional orientation for the image sensor.

One of the major drawbacks of using head-mounted devices with image sensors (and monitors) is that due to the many movements of the head (different angle, different distance) relative to an object being viewed, it is necessary to change the imaging settings very often as desired. Particularly in certain applications where it is not possible, or very difficult, to use the hands to adjust these sensors or screens in terms of parameters, it is not easy to find an efficient and good solution for this. For example, during surgical procedures there is a need to maintain sterility, and touching the device is therefore avoided, since the hands must in principle be made sterile again. Alternatively, another person can perform this so that the surgeon (or person in another position) does not have to compromise their sterility, but this presents other disadvantages such as the need for an extra person in the sterile space as well as the inconvenience and inefficiency of another person having to adjust specific settings for the wearer. In complex operations, there may not be a hand free to adjust the settings, and the same problem presents itself.

Often, however, it is necessary to frequently change perspective, and it is important (for example for remote users who follow along) that the image sensor does this under the correct parameters. If the user (or worse, another person) always has to adjust this themselves, this is accompanied by inconvenience, or it is simply impossible.

To this end, certain solutions can be provided, such as preset parameters that can be called up with the help of voice control or a movement (e.g. nodding), and then loaded automatically. However, in very complex exercises, this adds additional stress for the user, who has to find the right command that calls the right set of parameters. Moreover, in practice it is still difficult to find the "perfect" parameters.

In addition, it was noted that in many actions, a certain number of "standard situations" exist. These are fixed positions, orientations and perspectives that are repeated over and over, during every session. This is the case, for example, with medical actions, which are becoming increasingly standardized in order to create routines.

In this sense, the invention proposes to set and save certain sets of parameters, wherein these sets are stored associated with the specific orientation (and possibly also position) of the head-mounted device at the time the instructions to save are given (or at the time of saving itself). This ensures that these settings are correct, as they were in effect when the associated orientation/position was assumed.

By saving these preferences once in sets of parameters associated with the orientations, they can be used during each subsequent session, by automatically setting that associated set of parameters when recognizing the orientation (and possibly position). Not only is it possible in this way to significantly increase the efficiency in certain routines (such as operations), but in addition, a user can also save their own sets of settings under less common orientations (for example by tilting the head at a certain angle, automatic zoom), to call up and load them at desired times.

It should also be noted that further 'lock' commands may be provided, which can prevent the automatic loading of sets of parameters associated with an orientation, if it is desirable to retain a particular set of settings for a specified period of time. This can be done in different ways, e.g. via voice command, a certain movement or movement pattern or via a physical manipulation (button and the like).

In a preferred embodiment, the sensor system comprises one or more accelerometers, magnetometer, gyroscope and/or a similar sensor for detecting the orientation of the device. For example, one such sensor can be provided for each of the three main axes.

In some embodiments, the recognition of the orientation can be limited to a two-dimensional orientation. It is herein assumed that normal wearing of the device takes place in an upright position, looking straight ahead. For example, the two-dimensional orientation can comprise two angles from: the angle representing the deviation in the transverse plane (looking left or right); the angle representing the deviation in the sagittal plane (looking up, looking down); and the angle representing the deviation in the frontal plane (tilting the head). However, in a preferred embodiment, the three-dimensional orientation is used.

Thus, in a variation of the invention, the term "three-dimensional orientation" can be replaced by "two-dimensional orientation," usually referring to the orientation in the horizontal plane, and the invention can still be applied.

The same remark applies to the position. Preferably it will be the two-dimensional position (in the horizontal plane) which is used to characterize the "position" of the device, but in some embodiments the three-dimensional position can be used where more nuanced variations should be considered separately.

In a preferred embodiment of the invention, the method is configured to undo the step of automatically loading and setting the set of parameters by a predetermined action of the user of the head-mounted device, wherein the predetermined action may comprise a verbal command, may comprise a movement or movement pattern of the head-mounted device, and/or may be a tactile interaction of the user with the head-mounted device or with an electronic device coupled to the head-mounted device; preferably wherein undoing the loading and setting of the set of parameters comprises returning to the parameters upon recognizing the three-dimensional orientation.

The predetermined movement or movement pattern can be a specific movement that the user performs "consciously" (for example, nodding, shaking, making a circular figure, or other patterns, more complex or not), but it can also simply be "a movement" that exceeds the predetermined threshold values (or preferably exceeds these thresholds at least by a factor of two or greater, such as 3, 4, 5 or greater, to ensure that it is an actual movement).

In a preferred embodiment, the set of parameters associated with the recognized three-dimensional orientation is loaded and set after substantially maintaining the recognized three-dimensional orientation over a predetermined time of at least 2.0 seconds, preferably at least 2.5 seconds, or even 3.0 s, 4.0 s, or 5.0 s.

This condition avoids constantly loading new sets of parameters, as it is possible that during a movement to a certain orientation, several "stored" orientations are recognized, for which the parameters do not need to be loaded.

In a preferred embodiment, storing the set of parameters and associating them with the three-dimensional orientation of the head-mounted device is performed automatically upon detection of substantial maintenance of the three-dimensional orientation for a predetermined time of at least 5.0 seconds, preferably at least 10.0 s or even 15.0 s, 20.0 s or 30.0 s.

By automatically saving certain settings and associating them with an orientation upon maintenance of a certain position, it can be achieved that a user can save certain sets of parameters at any time, even without using their hands.

In a preferred embodiment, the wearer is informed that the parameters will be stored associated with a particular orientation. This can easily be done by a certain audio signal, or by a message, and/or by light signals, etc. That way, a user can abort the save if desired by taking an appropriate action (e.g., move head to abort).

In a preferred embodiment, the set of parameters stored is further associated with session characteristics, said session characteristics comprising one or more of: activity type, user type, user identity;
wherein the set of parameters is not loaded and set until the session characteristics apply in the current session.

Applying session characteristics ensures that by activity type (e.g. cataract surgery versus appendectomy) or by user (each with their own favorite settings, or even variations depending on length, which causes the orientation to vary) or by user type (for example, the specific role of a physician in a particular procedure may influence the routines that are needed and can be set, such as surgeon versus anesthetist, or assistant, etc.), the correct set of parameters is used.

Making use of this avoids having a huge plethora of "recognizable" orientations for which parameters can be loaded, and where with a small variation in posture you can accidentally end up in another, unwanted parameter setting.

In a preferred embodiment, the head-mounted device further comprises a sensor system for detecting the two-dimensional or three-dimensional position of the head-mounted device, absolute and/or relative to an external object, and storing the set of parameters associated with the two- or three-dimensional position of the head-mounted device upon confirming and/or storing the set of parameters, and wherein the set of parameters associated with a three-dimensional orientation and position is loaded and set upon the further condition of recognizing the two- or three-dimensional position.

On the basis of the absolute or relative position, a further distinction can be made between certain situations in which the user finds themselves. For example, by taking a step back from the operating table, they may wish to load a different set of parameters that are more relevant to that point of view. Including the positioning in the conditions to load a certain set of parameters thus makes it possible to store and dynamically load increasingly appropriate sets of parameters.

The relative position can be determined, for example, by means of a built-in element in the table, in the lighting or others, with which the head-mounted device can interact to determine a distance (for example via time-of-flight of the exchanged signal).

In a preferred embodiment, the steps of recognizing a three-dimensional orientation, and loading and setting the associated set of parameters may take place in a different session than storing the set of parameters.

In particular, it is useful to save sets of parameters for later use, i.e. in subsequent sessions. This way, the "repertoire" of saved sets of parameters can always be expanded, and these are saved across sessions.

In a preferred embodiment, the set of parameters stored includes at least the focal length, and preferably one or more of the following: film sensitivity of the image sensor, shutter speed, frames per second.

In a preferred embodiment, the head-mounted device comprises a display configured for displaying images from the image sensor.

In a preferred embodiment, the head-mounted device is a pair of smartglasses. However, alternative embodiments may also be provided in the form of a helmet, headset or headband, or as modular components attached thereto.

In a preferred embodiment, the head-mounted device comprises at least one display, controlled by the processor, wherein the method comprises a step of:
a. setting and confirming one or more settings under which the display operates during the session;
b. storing on the memory element all settings under which the display operates, comprising the set and confirmed one or more settings, wherein the stored settings are associated with the three-dimensional orientation of the head-mounted device upon confirming and/or storing all the settings;
c. upon recognizing a three-dimensional orientation of the head-mounted device as a three-dimensional orientation for which associated settings are stored, automatically loading and setting for the display the settings associated with the recognized three-dimensional orientation.

By also coupling the parameters for the display to certain orientations, the same advantage can be achieved as when using the coupled parameter sets for the image sensor.

In a preferred embodiment, the processor is provided on the head-mounted device, preferably smartglasses, as well as any components for wireless communication with other devices, for example via Wi-fi. An external control unit may still be provided that communicates wired or wirelessly with the head-mounted device, and optionally provided with further instructions, which are processed on the processor in the head-mounted device.

In an alternative preferred embodiment, the head-mounted device comprises an external control unit, which communicates wired or wirelessly with the head-mounted device, preferably wired, wherein the external control unit comprises the memory element and the processor, and wherein the external control unit is configured to control the image sensor based on information from, among others, the sensor system for detecting the three-dimensional orientation of the head-mounted device. Providing the processor unit on an external control unit or pocket unit (for example a mobile phone, smartphone, dedicated device for this, etc.) has the advantage that the weight of the head-mounted device is reduced. This also allows to outsource other matters, such as power supply, to the external control unit, which also has a strong influence on the weight of the device.

Preferably, the connection between the external control unit and the head-mounted device is wired, as this allows energy supply along the same connection, as well as saves weight because no wireless communication components have to be provided (which is often also less energy efficient).

Preferably, the display is configured to display the images recorded by the image sensor.

In a possible preferred embodiment, the components with processing power (processors and the like) are all provided on the external control unit, so as to reduce as much as possible the weight of the head-mounted device, as well as the heat generation that occurs with major operations. In addition, if the power supply is also from the control unit, this ensures that only a reduced part of the power has to go through the wired connection to the device, allowing a more efficient use of the energy, and also lower requirements for this cable.

Reducing the weight (and increased wearing comfort) of the head-mounted device is all the more important as the current application focuses on keeping the head still during the operation, wherein the holding still below a certain threshold of motion triggers the calibration of the operational parameters. Many actions where remote assistance/monitoring is relevant often take a long time, such as medical procedures. Certainly in such a case, it is crucial that a user can also act consciously during that long length of time, and that they can control the calibration in a targeted manner.

In an alternative embodiment, the device further comprises an external control unit, the external control unit being wiredly or wirelessly, preferably wiredly, in communication with the head-mounted device, and preferably comprising a processor unit for controlling the head-mounted device; wherein the external control unit comprises a power source for supplying power to the head-mounted device and wherein the head-mounted device does not comprise a power source.

In a preferred embodiment, multiple sets of parameters can be stored and associated for the same three-dimensional orientation.

This allows a user to use different sets of parameters for a given orientation, so that they can quickly and easily switch between these different perspectives, without forcing the user to assume a 'different' pose in order to load other sets of parameters.

In a preferred embodiment, upon setting and confirming new one or more parameters under which the image sensor operates for a three-dimensional orientation to which an already stored set of parameters is associated, the set of parameters associated with the new one or more parameters is stored as an alternate set parameters associated with the three-dimensional orientation. Upon recognizing a three-dimensional orientation as a three-dimensional orientation to which one or more alternative sets of parameters are associated, the user is notified thereof and can choose by means of predetermined commands between the already stored set of parameters associated with the three-dimensional parameters and the one or more alternative parameters associated with the three-dimensional orientation.

In a preferred embodiment, upon recognizing a three-dimensional orientation as a three-dimensional orientation to which multiple, mutually different, sets of parameters are associated, the user is notified thereof and can choose by means of predetermined commands between the multiple, mutually different sets of parameters associated with the three-dimensional orientation.

The predetermined commands can be verbal, via head gestures, hand gestures, etc., in order to choose a set of parameters, or to scroll through the available sets. By the presence of a display on the HMD, it's possible for the user to go through the available sets of parameters and choose the right one, in an efficient and quick manner.

In a preferred embodiment, the activity concerns a medical action, and in particular a medical procedure, such as an operation and the like. The invention is of course not limited thereto and can equally be used during a more regular doctor's visit. In addition, the activity may also concern maintenance, installation, removal work and the like for equipment, for example in hard-to-reach or dangerous places. Application in these aspects allows, among other things, that a remote user can follow everything by seeing the images captured by the sensor and, for example, giving instructions (whether or not on a screen of the device) to the user on site.

In a second aspect, the invention relates to a head-mounted device, preferably smartglasses, comprising at least one sensor system for detecting the three-dimensional orientation of the head-mounted device, an image sensor for capturing images, a memory element and a processor for controlling the image sensor, the sensor system and the memory element, wherein the device is configured to perform a method according to the first aspect of the invention, as described in this document.

In a third aspect, the invention relates to the use of the head-mounted device according to one or more of the embodiments according to the second aspect, by a medical professional (doctor, anesthetist, nurse, etc.) during a medical or surgical procedure, wherein the method according to the first aspect is carried out.

Further applications are, for example, in interventions on location (for example with an ambulance, but also police, technical interventions, fire brigade, etc.).

The benefits of a simplified control of a head-mounted device in this application are clear and have already been discussed, including preserving sterility, as well as avoiding an impact on wearer concentration.

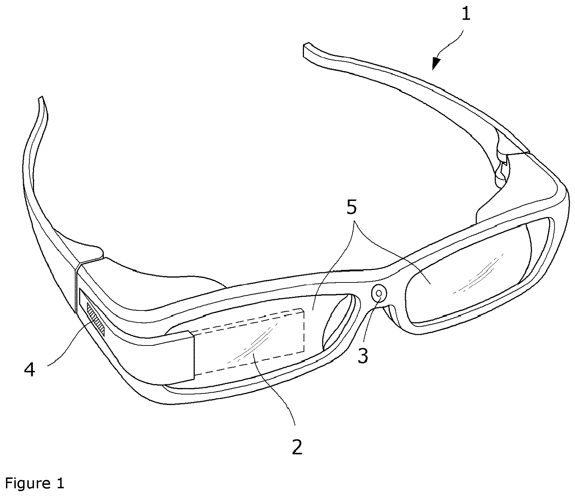

Figure 1 shows a possible embodiment of the invention, in the form of smartglasses (1), comprising a holder with a display (2), and wherein a camera or image sensor (3) is provided on the bridge between the two glasses (5). A number of electronic components (4) are incorporated in the glasses at various places, such as on the holder, but also in the arms of the glasses.

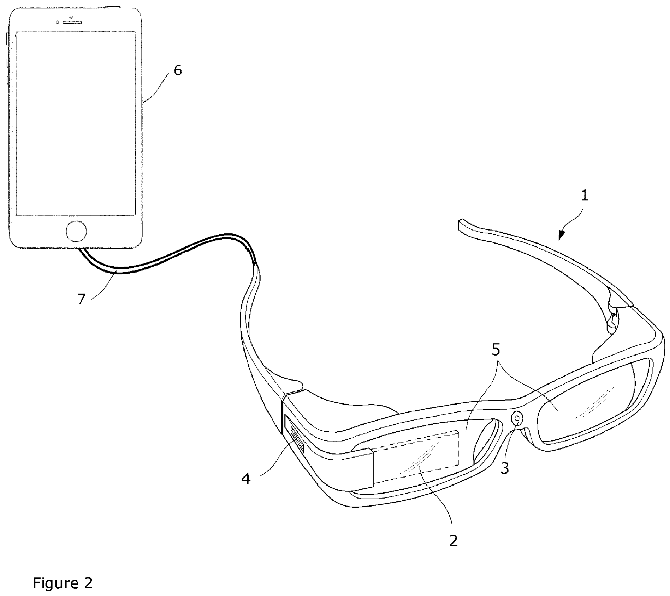

Figure 2 shows a possible variation on this, wherein a number of electronic components are provided in an external control unit (6), in this case a smartphone, which is wiredly (7) connected to the smartglasses (1). A number of the electronic components are no longer provided in the smartglasses, but their functionality is supported by the external control unit. This typically comprises power supply (battery), processor, etc.

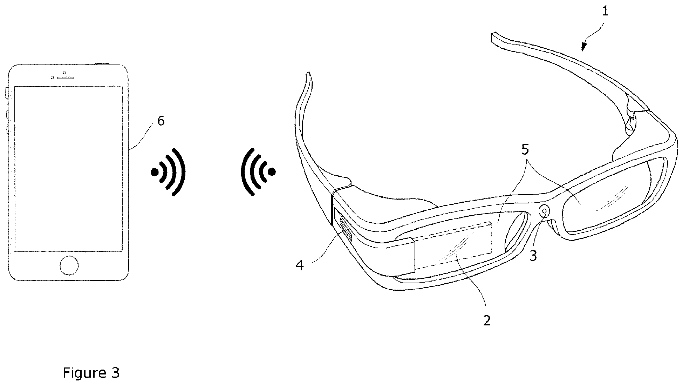

Figure 3 shows an adaptation of the version from Figure 2, wherein the communication between smartglasses and external control unit is wireless, by providing wireless communication components in the smartglasses and in the external control unit.

The present invention should not be construed as being limited to the embodiments described above and certain modifications or changes may be added to the examples described without having to re-evaluate the appended claims. For example, the present invention has been described with reference to medical procedures, but it should be understood that the invention can be applied to e.g. bomb dismantling or maintenance or repair works in areas difficult to access (aerial work platform, space station, Arctic facility, etc.).

## Claims

1. Method for using a head-mounted device in an activity, the head-mounted device comprising at least a sensor system for detecting the three-dimensional orientation of the head-mounted device, an image sensor for capturing images, a memory element, a display configured for displaying images from the image sensor and a processor for controlling the image sensor, the sensor system and the memory element,
the method comprising the following steps:
a. initiating a session of the activity;
b. setting and confirming during the session one or more parameters under which the image sensor operates;
c. storing on the memory element a set of parameters under which the image sensor operates, comprising the set and confirmed one or more parameters, wherein the set of parameters stored is associated with the three-dimensional orientation of the head-mounted device when confirming and/or storing the set of parameters;
d. upon recognizing a three-dimensional orientation of the head-mounted device as a three-dimensional orientation for which an associated set of parameters is stored, automatically loading and setting the parameters associated with the recognized three-dimensional orientation for the image sensor.

2. Method according to the preceding claim 1, the method being configured to undo the step of automatically loading and setting the set of parameters by a predetermined action of the user of the head-mounted device, wherein the predetermined action may comprise a verbal command, may comprise a movement or movement pattern of the head-mounted device, and/or may be a tactile interaction of the user with the head-mounted device or with an electronic device coupled to the head-mounted device; preferably wherein undoing the loading and setting of the set of parameters comprises returning to the parameters upon recognizing the three-dimensional orientation.

3. Method according to any of the preceding claims 1 or 2, wherein the set of parameters associated with the recognized three-dimensional orientation is loaded and set after substantial maintenance of the recognized three-dimensional orientation over a predetermined time of at least 2.0 seconds.

4. Method according to any of the preceding claims 1-3, wherein storing the set of parameters and associating them with the three-dimensional orientation of the head-mounted device is performed automatically upon detection of substantial maintenance of the three-dimensional orientation for a predetermined time of at least 5.0 seconds.

5. Method according to any of the preceding claims 1-4, wherein the set of parameters is stored further associated with session characteristics, said session characteristics comprising one or more of: activity type, user type, user identity;
and wherein the set of parameters is not loaded and set until the session characteristics apply in the current session.

6. Method according to any of the preceding claims 1-5, wherein the head-mounted device further comprises a sensor system for detecting the two- or three-dimensional position of the head-mounted device, absolute and/or relative to an external object, and storing the set of parameters associated with the two- or three-dimensional position of the head-mounted device upon confirming and/or storing the set of parameters, and wherein the set of parameters associated with a three-dimensional orientation and position is loaded and set upon the further condition of recognizing the two- or three-dimensional position.

7. Method according to any of the preceding claims 1-6, wherein the steps of recognizing a two- or three-dimensional orientation and loading and setting the associated set of parameters may take place in a different session than storing the set of parameters.

8. Method according to any of the preceding claims 1-7, wherein the set of parameters stored comprises at least the focal length, and preferably comprises one or more of the following: film sensitivity of the image sensor, shutter speed, frames per second.

9. Method according to any of the preceding claims 1-8, wherein the head-mounted device is a pair of smartglasses.

10. Method according to any of the preceding claims 1-9, wherein the head-mounted device comprises at least one display, controlled by the processor, wherein the method comprises a step of:
a. setting and confirming one or more settings under which the display operates during the session;
b. storing on the memory element all settings under which the display operates, comprising the set and confirmed one or more settings, wherein the stored settings are associated with the three-dimensional orientation of the head-mounted device upon confirming and/or storing all the settings;
c. upon recognizing a three-dimensional orientation of the head-mounted device as a three-dimensional orientation for which associated settings are stored, automatically loading and setting for the display the settings associated with the recognized three-dimensional orientation.

11. Method according to any of the preceding claims 1-10, wherein multiple sets of parameters can be stored and associated for the same three-dimensional orientation.

12. Method according to any of the preceding claims 1-11, wherein upon setting and confirming new one or more parameters under which the image sensor operates for a three-dimensional orientation to which an already stored set of parameters is associated, the set of parameters associated with the new one or more parameters is stored as an alternate set of parameters associated with the three-dimensional orientation; and wherein upon recognizing a three-dimensional orientation as a three-dimensional orientation to which one or more alternative sets of parameters are associated, the user is notified thereof and can choose by means of predetermined commands between the already stored set of parameters associated with the three-dimensional parameters and the one or more alternative parameters associated with the three-dimensional orientation.

13. Method according to any of the preceding claims 1-12, wherein upon recognizing a three-dimensional orientation as a three-dimensional orientation to which multiple, mutually different, sets of parameters are associated, the user is notified thereof and can choose by means of predetermined commands between the multiple, mutually different sets of parameters associated with the three-dimensional orientation.

14. Head-mounted device, preferably a pair of smartglasses, comprising at least one sensor system for detecting the three-dimensional orientation of the head-mounted device, an image sensor for capturing images, a memory element, and a processor for controlling the image sensor, the sensor system and the memory element, wherein the device is configured to perform a method according to any of the preceding claims 1-13.

15. Use of a head-mounted device according to claim 14, for the application of a method according to any of the preceding claims 1-13, by a medical professional in a medical treatment.
